Europäisches Patentamt

European Patent Office

Office européen des brevets

(11) Publication number: **0 373 800 B1**

# (12) EUROPEAN PATENT SPECIFICATION

(45) Date of publication of patent specification: **25.05.94**  (51) Int. Cl.[5]: **A61L 27/00**, A61F 2/32, C08L 23/06

(21) Application number: **89312565.8**

(22) Date of filing: **01.12.89**

(54) **Ultrahigh molecular weight linear polyethylene, articles and processes of manufacture.**

(30) Priority: **02.12.88 US 278913**
**22.12.88 US 288577**
**24.10.89 US 426916**

(43) Date of publication of application:
**20.06.90 Bulletin 90/25**

(45) Publication of the grant of the patent:
**25.05.94 Bulletin 94/21**

(84) Designated Contracting States:
**GR**

(56) References cited:

**POLYMER, vol. 22, January 1981, pages
23-28, IPC Business Press, London, GB;
BHATEJA: "Uniaxial tensile creep behaviour
of ultra high molecular weight linear poly-
ethylene"**

**JOURNAL OF POLYMER SCIENCE, part A2,
Polymer Physics, vol. 7, 1969, pages
2051-2059, John Wiley & Sons, New York, US;
DAVIDSON et al.: "Extended-chain crystals.
II. Crystallization of polyethylene under ele-
vated pressure"**

(73) Proprietor: **E.I. DU PONT DE NEMOURS AND
COMPANY
1007 Market Street
Wilmington Delaware 19898(US)**

(72) Inventor: **Howard, Edward George Jr.
844 Old Public Road
Hockessin, Delaware 19797(US)**

(74) Representative: **Jones, Alan John et al
CARPMAELS & RANSFORD
43 Bloomsbury Square
London, WC1A 2RA (GB)**

EP 0 373 800 B1

**Description**

BACKGROUND OF THE INVENTION

1. Field of the Invention

This invention relates to a novel ultrahigh molecular weight linear polyethylene (UHMWLPE). This novel UHMWLPE, in the form of a shaped article, exhibits in various embodiments a unique combination of properties making the material useful as a bearing surface, in general, but particularly useful as a prosthetic hip joint cup and as other prosthetic shapes for replacement of other joints of the human body.

2. Description of the Prior Art

In U.S. Patent No. 3,944,536 (March 1976), Lupton et al describe UHMWPE in the form of a fabricated article exhibiting an elastic modulus of 340,000 to 500,000 psi, a tensile impact strength of 140 to 600 ft lb/in$^2$, a density of 0.95 to 0.98 g/cc at 25°C, a crystalline melting point of 142 to 148°C (as measured by differential thermal analysis) and a unique crystalline form characterized by the absence of fold spacings of 50-2000 Angstrom units (Å) and the presence of crystal spacings of about 10,000 Å. The critical feature of the process of producing this UHMWPE is disclosed to involve inducing crystallization of the molten polymer above 150°C by rapidly increasing the applied pressure from an initial level of 1 to 1000 atmospheres to a second level of 2000 to 7000 atmospheres and then cooling rapidly while maintaining a pressure sufficient to maintain the polyethylene in the solid phase until the temperature is below the crystalline melting point of the polyethylene at atmospheric pressure.

In Kunstuffe German Plastics 77 (1987) pp. 617-622, in an article entitled "Ultrahigh Molecular Polyethylene for Replacement Joints", Eyrer et al. point out that the service life of joint replacements made of UHMWPE is limited. Analysis of the damage to over 250 explanted hip cups and tibial plateaus revealed a changed property profile which they explained by post-crystallization resulting from oxidative chain decomposition. They suggested optimizing the processing of polyethylene under higher pressure and higher temperature to increase the degree of crystallinity. The Eyrer et al. product displays a creep of above 5% at a compression of 1000 psi (6.9 N/mm$^2$) for 24 hours at 37°C.

One of the most remarkable advances in the medical field in recent years is the development of prosthetic joints, particularly the load bearing hip. The crippled and sometimes bed ridden elderly can walk again. The key to this development is UHMWPE because, not only does it have the necessary impact strength, but it initiates no adverse blood reactions. But at present, these prosthetic joints are limited to the older, less active segment of the population because the polymer tends to creep under the pressure that a younger more active person might develop while involved in recreation or employment. The creep would cause the loss of the close tolerance required between the plastic socket and the polished metal ball attached to the femur. These changes in dimensions disturb the distribution of walking forces which in turn accelerates more creep and wear. Eventually the increased pain requires a traumatic revision operation. One objective of this invention is to provide UHMWPE prosthetic joints with improved creep resistance hence removing some of the age restriction existing on the present polyethylene joints.

SUMMARY OF THE INVENTION

The object of this invention is to provide a tough UHMWLPE composition and article with creep resistance that is less than 1% (as measured at a temperature of 23 ± 1°C and a relative humidity of 50 ± 2% for 24 hours under a comparison of 1000 psi) and that maintains excellent tensile and flexural properties.

Specifically, the product of this invention is a composition exhibiting an elastic or flexural modulus of 250,000-500,000 psi (1724 - 2488 MPa), a tensile stress at yield of 3500-4500 psi (24.1 - 31.0 MPa), a tensile stress at break of 4000-9000 psi (27.6 - 62.1 MPa), a tensile modulus 300,000-700,000 psi (1724 - 4827 MPa), preferably 300,000-600,000 psi (2069 - 4137 MPa), an elongation of 200-500%, a notched Izod impact resistance of 12-25 ft. lb. per in. (641 - 1335 Nm/m) of notch, a creep at a compression of 1000 psi (6.9 MPa) of less than 1% after 24 hours at a temperature of 23°C and a relative humidity of 50%, the polyethylene having a molecular weight of 1,000,000-10,000,000 (the molecular chain length between folds being greater than 3500Å), a single crystalline melting point of greater than 144°C (as measured by differential scanning calorimetry) the reduction in said melting point upon reheating being greater than 11°C above that of the starting polymer and an infrared crystallinity index of at least about 0.45, preferably at least 0.5. The composition may be formed into an article.

The process for obtaining the article of this invention involves six (6) important steps:

1. forming, by milling or casting or the like the article from UHMWLPE having a molecular weight of 400,000-10,000,000, preferably at least 1,000,000 and most preferably at least 6,000,000;

2. placing the article in a pressure vessel (e.g. an autoclave) substantially filled with a fluid that is inert to the article, preferably water;

3. heating the vessel to a temperature of at least 190°C, preferably 200°C-230°C, and raising the pressure in the vessel, usually by adding additional water, to at least 2800 atmospheres (ATM) (280 MPa), preferably at least 3000 ATM (300 MPa);

Alternatively, the article, water, and reactor are heated to about 200-230°C, the pressure is applied and the system held at this temperature while polymer slowly loses heat of crystallization and solidifies.

Also, the pressure can be applied at any temperature during the heating cycle if the temperature exceeds about 200°C.

4. maintaining the temperature and pressure substantially as selected in step 3 for at least 0.5 hour, preferably at least one hour;

5. thereafter, cooling by reducing the temperature to a temperature at least below about 160°C-170°C preferably to 160°C or below, most preferably below 140°C, while maintaining a pressure of at least 2800 ATM (280 MPa) preferably at least 3000 ATM (300 MPa) at a slow rate, the rate of cooling being such that temperature gradients in the shaped article are substantially avoided. The polymer must be cooled slowly at the high pressure until it is fully crystallized. At 3000 ATM (300 MPa) pressure, the crystallization temperature of UHMWLPE of over one million molecular weight is in the range of 170°C-190°C. The pressurized vessel should be cooled slowly to insure that the temperature of the polymer is not significantly above the vessel temperature, particularly if the pressure vessel construction does not permit means for measuring the temperature of the polymer itself.

6. cooling and releasing the pressure on the shaped article in a manner such that any remelting of the article is prevented. This is accomplished by cooling at least to a temperature below the atmospheric pressure melting point, i.e., about 130°C-135°C preferably below 120°C, most preferably below 100°C and releasing the pressure to reduce it from at least 2800 ATM (280 MPa) to approximately 1 ATM (0.1 MPa), either sequentially or simultaneously. It should be understood that it is necessary to cool the polymer to a temperature below its melting point at any particular pressure to ensure that none of the polymer melts as the pressure is reduced since lowering the pressure lowers the melting point.

As an optional seventh step, it is advisable to shave the surface of the article, i.e. remove approximately the outer 2 millimeters that might contain any fluid-affected polymer.

A very important step is the fifth step, i.e. cooling in a manner that limits severe temperature gradients in the article. For example, for a 1 inch X 6 inch rod (25mm x 150mm), a cooling rate of approximately 10°C per hour is usually necessary. Although cooling rates no greater than 10°C per hour are preferred, cooling rates as high as about 35°C per hour have been used to provide the product of this invention. However, these latter rates require careful control in order to limit temperature gradients during cooling. Cooling rapidly, as taught in the prior art, will not provide the article of this invention. This invention is particularly useful for manufacturing shaped articles where temperature gradients pose a problem during the cooling step, i.e., where the article's cross-sectional dimensions are at least 1 inch x at least 1 inch (25mm x 25mm), usually for joints at least 1 inch x at least 2 inches (25mm x 51mm) Specifically, the importance of this step and of this invention is manifest in producing articles having as its smallest dimension 0.2 inch (5mm), i.e., at least 0.2 inch (5mm) in thickness. It has been found that in such articles, the temperature gradients must still be controlled by the process of this invention in order to obtain the product of this invention.

Alternatively, for steps 2 and 3, the pressure vessel can be used more efficiently by preheating polymer outside the reactor because heating polyethylene is a very slow process due to its high heat of fusion and its thermal insulation property. It can be heated in an oven, preferably in an inert atmosphere to prevent oxidation, particularly when the tempereature is above 160°C. Because the UHMW polyethylenes do not flow when melted, they can be handled while hot and transferred to the preheated pressure vessel without deforming.

Product resulting from this modified process also is excellent for orthopedic replacement and has certain superior characteristics. Values demonstrating these superior, enhanced qualities are shown in Example 11.

In addition to utility in the field of orthopedic replacement, the products prove useful in other applications also requiring the special properties of the products. Not only shaped articles are of interest, but also films and fibers as well as other "downstream" forms and unshaped granular forms of the product will prove useful. Film formed of the product of Example 5 is exemplified in Example 13. These examples are illustrative only, and other forms, shaped and unshaped, of the composition are contemplated within the scope of the invention. Therefore, "article" shall include both shaped articles and unshaped articles.

In a process more recently developed for manufacturing the product of this invention, the fluid used in the pressure vessel could be a gas that does not affect the UHMWLPE adversely. Specifically, the shaped article is formed from commercially available UHMWLPE. In the second step, the article is placed in a pressure vessel containing the fluid, in this case argon. However, it is necessary in the case where argon is used, to protect the UHMWLPE from any entry of the gas into the polymer by surrounding the article with a thin stainless steel or similar metal can as described in copending U.S. Patent Application (DE-0289C) filed to S. Li on November       , 1989 which is a continuation-in-part of U.S. Serial No. 07/426,918 filed on October 24, 1989 which in turn is a continuation-in-part of U.S. Serial No. 07/288,576 filed December 22, 1988 which in turn is a continuation-in-part of U.S. Serial No. 07/278,912 filed on December 2, 1988. It should be understood that other gaseous fluids may be used in place of argon. So long as the gas does not adversely affect or is prevented from adversely affecting the polymer and is not affected by the temperatures and pressures used in the process, the gas may be used.

In the next step, a pressure of at least 2800 ATM is applied and the vessel is heated to about 220°C for about 6 hours. Thereafter, the temperature is "ramped" down at a rate no greater than about 10°C per hour to about 160°C while maintaining the pressure above 2800 ATM (280 MPa). The temperature is then "ramped" down at a maximum rate to 50°C while maintaining the high pressure, after which the pressure is released.

A further embodiment of the invention involves the following important steps:

1. forming by milling a casting or the like, the article from UHMWPE having a molecular weight of 400,000-10,000,000, preferably at least 1,000,000;

2. subjecting said article to a temperature of 190-340°C, preferably 320-340°C, for at least 0.5 hour, preferably at least one hour, in an inert atmosphere; and

3. cooling the article non-precipitously to a temperature of about 130°C or below. As in the other embodiments, the rate of cooling is such that temperature gradients in the shaped article are substantially avoided. Again, the rapid cooling sought in the prior art will not provide the article of this embodiment. The product of this non-pressurized embodiment is particularly useful for manufacturing shaped articles where temperature gradients pose a problem during the cooling step, i.e., where the article's cross-sectional dimensions are at least 1 inch x at least 1 inch (25mm x 25mm), usually for joints at least 1 inch x at least 2 inches (25mm x 51mm). Specifically, the importance of this step and of this invention is manifest in producing articles having as its smallest dimension 0.2 inch (5mm), i.e., at least 0.2 inch (5mm) in thickness.

For purposes of this invention, ultrahigh molecular weight linear polyethylene (UHMWLPE) is defined as a linear polyethylene having an estimated weight-average molecular weight in excess of about 400,000, usually 1,000,000 to 10,000,000 as defined by a melt index (ASTMD-1238) of essentially zero and a reduced specific viscosity (RSV) greater than 8, preferably 25-30. The relationships of RSV to intrinsic viscosity and to molecular weight are those developed by R. Chaing as presented by P. S. Francis et al. in J. Polymer Science, 31, 453 (1958).

It is envisioned that the modifications of adding a preliminary step heating the product to 320-340°C will also provide superior characteristics to the product described by Li.

The improved properties of the products of this invention in its various embodiments are reflected in a tensile modulus of at least 300 kpsi (2069 MPa), a flex modulus of at least 250 kpsi (1724 MPa), ultimate tensile strength greater than 4000 psi (27.6 MPa), yield strength greater than 3500 psi (24.1 MPa) and an elongation at break no greater than 500%.

A very important property of the product of this invention is its creep resistance. For prosthetic devices, e.g. knee, hip, elbow joints, etc., any substantial creep can be devastating in the loss of the benefits of extremely expensive surgery. Thus, the shaped articles of this invention display as little as a 0.5% loss in thickness when subjected to a compression pressure of 1000 psi (6.9 MPa) for 24 hours at a temperature of 23°C and a relative humidity of 50% in accordance with ASTM D-621.

For some applications, still lower creep, higher stiffness, higher elongation, and particularly higher tensile strengths at yield are necessary. These can be introduced by the enhancing process if the polyethylene is first thermally heated at 320-340°C for thirty minutes or longer. The process depends on heating the polymer as near as possible to, without reaching, its decomposition point. The hot polymer should be cooled slowly because very rapid cooling, such as immersion in cold water, causes internal voids to form. This is caused by the combination of large volume changes polyethylene undergoes when melting (about 30%) and its poor heat conductivity. A convenient method is to allow the polymer to cool wrapped in insulation. The hot polyethylene can be put directly into the hot pressure vessel or it can be cooled and reheated to the normal 200°C in the pressure vessel.

The improved properties of the product of this part of the invention are reflected in tensile modulus of at least 350 kpsi (2413 MPa), a tensile strength at yield of 3500-5400 psi (24.1 - 37.2 MPa), and a creep value of less than 0.6%.

In addition, the thermally heated polymer, still in the folded chain form used to make this superior form of this invention, in itself has improved elongation, crystallinity, and impact resistance over the starting material, but it is not equivalent to the enhanced form of polyethylene.

Perhaps the most characteristic property of the product of this invention is its infrared crystallinity index (IRCI). This property, which provides a reasonably accurate reflection of the crystallinity of this material, is in a range never before attained with any UHMW polyethylene materials. To determine this index, samples are first obtained by microforming thin sections. Heat and pressure should be avoided during preparation of the samples. ICRI is the ratio of the band at 1894 reciprocal centimeters ($cm^{-1}$) to the band at 1305 reciprocal centimeters ($cm^{-1}$). Since the band at 1894 $cm^{-1}$ is attributed to the crystalline nature of the material and the band at 1305 $cm^{-1}$ is attributed to its amorphous nature, ICRI increases as the crystallinity increases. The product of this invention displays an IRCI of at least about 0.45. In fact, values of 0.73 and higher have been obtained. On the other hand, IRCI values for prior known UHMWLPE's seldom reach above 0.3.

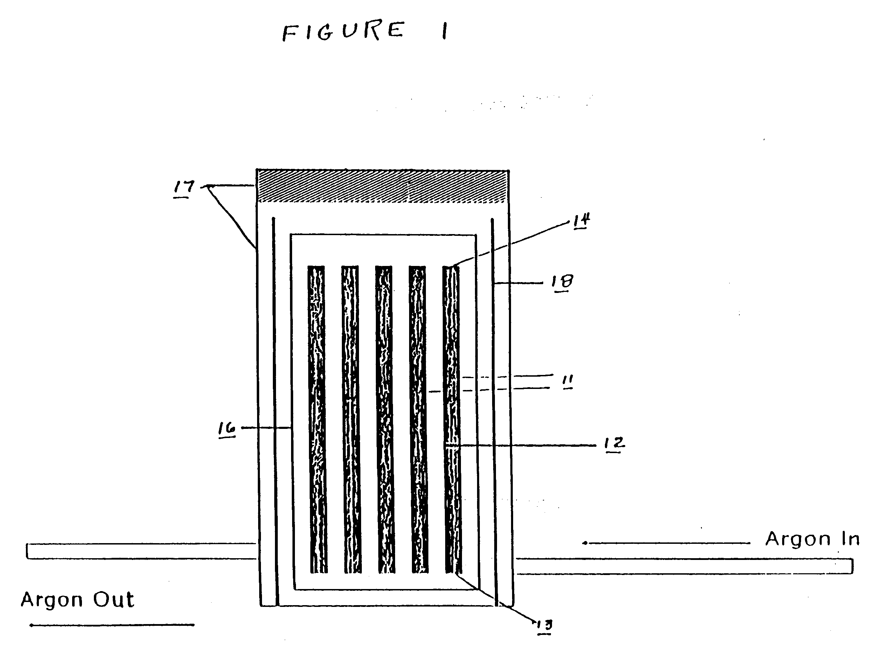

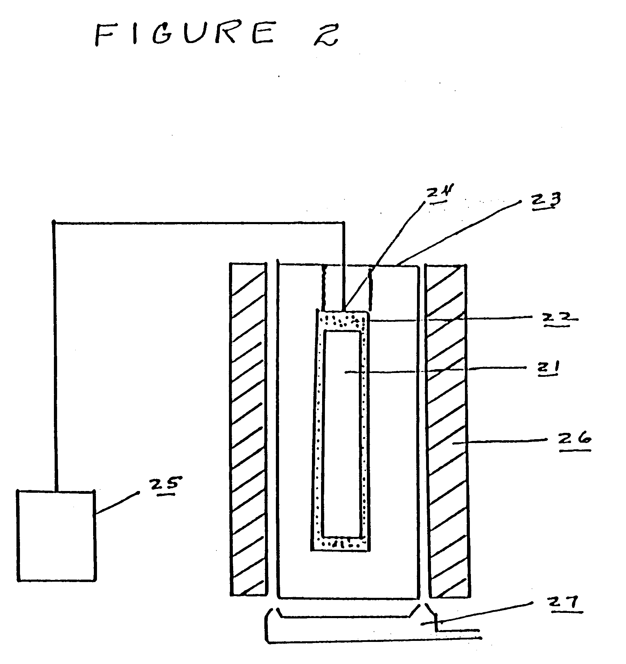

The invention will be more clearly understood by referring to the drawings and examples, which follow. In the drawings, Figure 1 is a schematic diagram of the equipment used in the process for forming the product of the invention using argon gas; and Figure 2 is a schematic diagram of the equipment used in the hydrostatic process.

In the examples, most of the properties are measured using standard ASTM tests.

All of the physical measurements were carried out under constant humidity (50% relative humidity) and temperature (23°C) conditions.

Tensile modulus, ultimate tensile strength, yield strength and elongation were measured according to ASTM D-638 with the following modifications:
- samples machined into shape without lubricating fluid
- type I tensile bar
- cross head speed = 0.2"/min (5mm/min) for tensile modulus
      2.0"/min (51mm/min) for tensile stress and elongation.

Creep resistance was measured in accordance with ASTM D-621 with the following modifications:
- samples machined into cylinders or cubes without the use of lubricating fluids
- samples measured 0.5" x 0.5" x 0.5" (13 x 13 x 13mm)

Flexural properties were measured according to ASTM D-790 with the following modifications:
- samples machined into shape without the use of lubricating fluids
- typical flex bar measures 0.125" thick x 0.5" width x 5" length (3 x 13 x 127mm)
- span or gauge is 2.0" (51mm). (This was determined by a span/depth ratio of 16/1.)
- cross head speed = 0.05"/min (1mm/min) (calculated based on span).

Impact resistance was measured using the notched Izod test given in ASTM D-256 with the following modifications:
- samples machined into shape without the use of lubricating fluid
- type A or notched IZOD
- specimen size is 0.5" x 2.5" (13 x 64mm)
- 0.4" from bottom of vertex to opposite side
- 1.25" (32mm) impacted end (from end of bar to vertex of notch)
- the notch should be the specified angle of 22.5 degrees.

It should be appreciated that in all embodiments of the invention the step of forming the article by milling, casting, or the like from UHMWLPE may be performed as the first step in the process (i.e., before heating or preheating) or as the last step in the process (i.e., after the cooling step).

The following non-limiting examples, including the improved and superior embodiments, illustrate the basic principles and unique advantages of the present invention. Various changes and modifications may be made without departing from the spirit and scope of the present invention.

## EXAMPLE 1

The material used in this example is American Hoechst Hostalen 415 GUR ultrahigh molecular weight polyethylene. It was obtained in the form of bars, 3" in diameter and up to 5' long in length (76mm to 1.5m). The material will be referred to as UHMWLPE. The molecular weight was over 1,000,000.

One or more pieces of the UHMWLPE 11 were placed into stainless steel, seamless, 48" (1.22 m) long cylinders or sleeves 12. The thickness of the stainless steel was 1/8" (3mm). The bottom of the cylinders was closed by welding a stainless steel cap 13 onto the bottom of the cylinder. The top of the cylinder was partially closed by welding on a modified cap 14 which contained a vacuum port 15. The cylinder was then evacuated using a vacuum pump and sealed by crimping the port to form a can that surrounds the piece of UHMWLPE completely. The sealed cylinder was then placed in a containment vessel 16 large enough to hold 15 cylinders. The containment vessel 16 was then placed into a hot isostatic pressing (HIP) unit 17 with molybdenum heating units 18. Thermocouples were added to monitor the temperature of the cylinders.

The basic function of the HIP process is to uniformly heat a load while applying pressure uniformly to all surfaces. The pressure medium used in this case was argon. The UHMWPE is protected from contact with the argon by the stainless steel cans.

The process conditions were:

1. Apply pressure to 39,000 psi. (269.1 MPa)

2. Heat to 220°C.

3. Hold for 6 hours at 220°C and a minimum pressure of 41,000 psi (282.9 MPa) (27 atmos.) (2.7 MPa).

4. Ramp temperature down at a rate no faster than 10°C per hour to 160°C. Pressure is maintained above 41,000 psi (282.9 MPa) during this time.

5. Ramp temperature down at maximum rate to 50°C while maintaining the pressure above 41,000 psi (282.9 MPa)

6. Below 50°C, pressure may be let down and the cycle ended.

The UHMWPE rods were then removed from the sleeves and parts were fabricated for physical testing. It is noted that the material produced exhibits much higher tensile modulus, flex modulus, melting point, density and creep resistance than the starting material (Control A).

| Material | DSC Melting Point (°C) | Density (grams/cc) | IRCI |
|---|---|---|---|
| Control | 137.0-140.7°C | .93-.94 | 0.24 |
| Example 1 | 148.0-152.0°C | .947 | ≥ 0.45 |

| ASTM D638 | Control A | Example 1 |
|---|---|---|
| Flex Modulus kpsi (MPa) | 165 (1138) | 291 (2006) |
| Tensile Modulus kpsi (MPa) | 185 (1276) | 315 (2172) |
| Ultimate Tensile kpsi (MPa) | 4500 (31028) | 4688 (32324) |

ASTM D638

| | | |
|---|---|---|
| Yield kpsi (MPa) | 3476 (23967) | 4082 (28145) |
| Elongation (break) % | 262 | 227 |

[values are averages of 5 tests]

ASTM D621 Creep Test

Load

| | | |
|---|---|---|
| 500 psi (3.45 MPa) | .5 | .3 % deformation |
| 1000 psi (6.9 MPa) | 1.6 | .7 |
| 2000 psi (13.8 MPa) | 5.9 | 2.4 |

Additional evidence of the products' distinctiveness is found in data produced by small angle X-ray testing. A truly characteristic small-angle X-ray scattering plot of desmeared intensity (by the method of P. W. Schmidt, Acta Cryst., 13, 480 (1960) and Acta Cryst., 19, 938 (1965)) (I x (2 theta) squared) versus scattering angle (2 theta) for the material of the invention exhibits two distinct scattering peaks associated with crystal long-spacings in the range of 480 angstroms (at 2 theta = .184 degrees) and 4610 angstroms (at 2 theta = .0192 degrees). The presence of the sharp diffraction peak at the lower angle is indicative of an extended polymer chain conformation (with a lamellar thickness greater than 2000 angstroms) whereas the more diffuse higher-angle peak corresponds to a lamellar thickness characteristic of conventional folded chain PE. This provides clear evidence for the presence of two scattering peaks in the subject invention material which correspond to lamellar thicknesses both above and below 2000 angstroms. By comparison, the previously patented extended chain polyethylene of Lupton et al., was reported to exhibit a complete absence of any detectable small angle X-ray scattering in the range of 50 to 2000 angstroms. Consequently this work demonstrates that the subject invention material is morphologically distinguishable from Lupton et al.

EXAMPLE 2

The material used in this example is an ultrahigh molecular weight polyethylene obtained from Jet Plastics, Inc.

A rod 21 measuring 6" x 1 1/8" (152 x 32mm) was placed in the cavity 22 of a stainless steel, seamless, cylindrical reactor 23. The cavity 22 had a diameter of 1.35" (34mm) and was about 9" (23mm) long.

Water was fed into the cavity 22 at the entry port 24 through the use of a high pressure water pump 25 powered by compressed air. Simultaneously, the reactor was heated by electrical heaters 26 surrounding the reactor.

In the first step, the rod 21 was heated to a temperature of 220°C under a hydrostatic pressure of 2000 ATM (200 MPa). The pressure was raised to 3000 ATM (300 MPa) while the temperature was maintained at 220°C for 2 hours. The temperature was permitted to fall to 209°C over another 2 hour period, and then to about 182°C in 4 hours. Finally, the rod was cooled to 49°C by subjecting the reactor 23 to compressed air from the blower 27 over a period of one hour and the pressure released.

The rod was removed from the reactor and the surface was shaved. The product, a sample taken substantially from the center of the rod, displayed a DSC melting point of 154.5°C, and, on reheating, a DSC melting point of 140°C.

The material, when subjected to a compression pressure of 1000 psi (6.9 MPa) for 24 hours at a temperature of 23°C and a relative humidity of 50% in accordance with ASTM D-621, deformed only 0.4%.

The other properties of the product were:

| flexural modulus | - over 250 kpsi (1724 MPa) |
| tensile modulus | - over 300 kpsi (2068 MPa) |
| tensile stress (yield) | - over 3500 psi (24.1 MPa) |
| tensile stress (break) | - over 4000 psi (27.6 MPa) |
| elongation (break) | - less than 500%. |

Its infrared crystallinity index was over 0.5.

The hydrostatic process described in Example 2 is the best mode for preparing the product of this invention. This process has important advantages. The pressure transfer liquid, water, is non-toxic and inexpensive. The hydraulic pressure is applied equally in all directions resulting in a substantially homogeneous product. This compares to processes shown in the prior art where hydraulic pressure is applied by a piston or a ram. In these latter cases, the high shrinkage polymer tends to solidify along the heat-escaping walls making it difficult for the pistons to advance and still apply the pressure uniformly. The result is a heterogeneous product.

It should be understood that although water is the preferred liquid fluid to use in the process, other liquids, with the same criteria as mentioned for gases, are also useful. Thus, methanol, ethanol, glycerin, glycols, etc. in addition to various aqueous solutions may be used.

The salt selected for an aqueous solution may be one that imparts a desirable property to the surface of the shaped article.

EXAMPLE 3

This experiment was carried out in a manner similar to Example 2 except that the pressure in the first step was 3000 ATM (300 MPa). The material was maintained at 220°C under 3000 ATM (300 MPa) for 4 hours. The temperature was allowed to fall to 190°C over an 8-hour period. After which, it was cooled to 100°C in 1 hour.

Samples were taken from 1/8" (3mm) inside both ends of the rod and had melting points of 150.8°C and 153.2°C. When reheated, the melting points were 135.5°C and 138°C, respectively.

The infrared crystallinity index was 0.791; and the creep, when measured in accordance with ASTM D-621, was less than 1%. These measurements were obtained on a sample taken from the center of the rod.

EXAMPLE 4

The experiment was also carried out in a manner similar to Example 2 except for the following changes in the heating/cooling cycle:

EP 0 373 800 B1

Heat at 211°C and 3000 ATM (300 MPa) and maintain for 1 hour;

Cool to 200°C in 1 hour at 3000 ATM (300 MPa);

Cool to 180°C over 5 hours at 3000 ATM (300 MPa) (cooling rate 200→180°C, 4°/hour); and

Cool to 33°C in 1 hour and 3 minutes.

The product from inside both ends melted at 150°C and on reheating, at 135.5°C. The product, when tested in accordance with ASTM D-621 displayed a creep of less than 1%. Its infrared crystallinity index was 0.652.

EXAMPLE 5

A reactor with the general configuration shown in Figure 2 having an internal diameter of 4" (102mm) and being 22" long (559mm), was charged with a 3 1/8" x 18 1/16" (79 x 459mm) rod of UHMWPE (made from polymer from American Hoechst, Hostalen GUR 415). The closed vessel was evacuated, filled with water, and heated to 232°C at which point the pressure was increased to 3000 ATM with the water pump. This pressure was maintained until the end of the experiment. The reactor was held between 210 and 230°C for 3 hours, cooled over 1 hour to 200°C, cooled to 175°C in 5 hours (5°/hour) and then cooled to 80°C in 7 1/2 hours.

The resulting product rod was still in a cylindrical form with very little warpage. It measured 3 1/8" x 17 15/16" (79 x 456mm). End pieces, 1/2" (13mm) thick, were cut off each end of the rod revealing a uniform white color. Samples taken from the center of the rod on these cuts gave melting points of 152.9°C (201 J/g) and 152.1°C (207 J/g) when heated at 20°C/minute. When reheated, the melting points were 137.5°C.

A six inch (152mm) section of the rod was sawed into 3/16" (5mm) thick shapes for physical tests, then carefully milled to remove saw marks to 1/8" (3mm) thickness. The resulting polymer had the following properties:

| IZOD | 18.7 ft. lb./in. of notch (998 Nm/m) |
| Flexural Modulus | 298.9 kpsi (2061 MPa) |
| Tensile Properties | |
| Stress at yield | 4190 psi (28.9 MPa) |
| Stress (max.) (at break) | 5430 psi (37.4 MPa) |
| Elongation (at break) | 280% |
| Modulus | 378.3 kpsi (2608 MPa) |
| Creep Test, 1000 psi | 0.6% |

All tests at room temperature.

The crystallinity index (IRCI) was 0.528.

EXAMPLE 6

In this example, the product was prepared with an exceedingly smooth surface.

A polished brass disk, about 1 1/2" (38mm) diameter, 1/4" (6mm) thick was pressed at 160°C against a UHMW polyethylene plug. The combination was cooled under pressure and sealed in a heat shrinkable Teflon FEP™ tube. The polyethylene was converted in a hydrostatic system by this procedure in the vessel used in Example 5.

The heating, cooling cycle was as follows:

3000 ATM and 210°C in 1 hour;

3000 ATM 210°C to 200°C in 1 hour;

3000 ATM 200°C to 178°C in 6 hours, 45 minutes; and

3000 ATM 178°C to 23°C in 2 hours, 20 minutes.

(3000 ATM = 300 MPa)

The polyethylene did shrink so that it had a smaller diameter than the disk, but the polymer stuck to the surface. When forced apart, the surface was extremely smooth.

This technique is important in preparing complicated surfaces where smoothness is extremely important, such as on bearing surfaces such as medical prosthesis for knee and hip joints, or bearings for motor shafts, etc. Machine cutting polymers always leaves very small ridges.

9

EXAMPLE 7

The reactor of Figure 2, internal diameter 4" by 22" long was charged with a 3" x 18" (76mm x 457mm) rod of American Hoescht, Hostalen GUR 415 ultrahigh molecular weight polyethylene, water, and a nominal pressure of 100 psi (7 MPa). The system was heated to 170°C to 176°C and held there for 1 hour, then the pressure was raised to 3000 ATM (300 MPa). The temperature was maintained at 179°C-174°C for 3 hours, during which time the polyethylene crystallized. The reactor was cooled to 79°C in 1.7 hours.

Two samples were taken; one from the center of the rod and another 1/2 inch (13mm) from the outer surface of the rod. The melting points, as measured by DSC, were 150.9°C and 150.4°C, respectively, and upon reheating, 136.6°C and 137/3°C. Thus, the increases in melting points were 14.3°C and 12.7°C, respectively. The infrared crystallinity index was 0.5.

EXAMPLE 8

This example shows that the polymer can be cooled at a rate as high as 34.5°C per hour in the critical cooling step (step 5) if proper precautions are taken to limit temperature gradients.

A one inch rod of UHMWLPE from Jet Plastics, Inc. was used. It was placed in the pressure vessel with water and subjected to the following treatments:

3000 ATM and 220°C for 2 hours;
3000 ATM, cool to 200°C in 50 minutes;
3000 ATM, cool to 177°C in 40 minutes;
3000 ATM, cool to 35°C in one hour.
(3000 ATM = 300 MPa)

A test sample taken one-half inch from the end of the rod and in the center displayed a DSC melting point of 153.8°C, and, on reheating a DSC melting point of 139.7°C.

The material, when subjected to a compression pressure of 1000 psi (6.9 MPa) for 24 hours at 23°C and a relative humidity of 50% in accordance with ASTM D-621 deformed 0.5%.

EXAMPLE 9

Superior Enhanced UHMW Polyethylene prepared by Preheating Polymer to 325°C.

A 3-1/16" x 15" (78 x 381mm) rod of UHMW polyethylene (Hoechst GUR415, fabricated by PolyHi) was heated to 325°C in an atmosphere of $N_2$ for six hours. The hot rod was quickly placed in a pressure vessel preheated to 212°C. The vessel was sealed immediately and pressured with water to 3000 ATM. The cooling schedule was as follows:

212° to 191°C 65 minutes
191° to 181°C 63 minutes
181° to 175°C 2 hours
175° to 174°C 6 hours, 26 minutes
174° to 45°C 3 hours, 15 minutes

The rod was cut into test samples and analyzed with the following results:

DSC (Differential Scanning Calorimetry)

|  | Center of Bar | 1 cm from Bar Edge |
|---|---|---|
| m.p., °C | | |
| 1st heat | 150.5 | 152.4 |
| 2nd heat | 137.9 | 139.0 |
| $\Delta T$ | 12.6 | 13.4 |

Heat of Fusion

| 1st heat | 198.8 J/g |
|---|---|
| 2nd heat | 134.4 J/g |

Infrared Crystallinity Index

(Samples cut from within 5 mm of bar edge)

| In Bar Direction | 0.613 |
|---|---|
| Perpendicular to Bar Direction | 0.633 |

Flex Modulus (KPSI)

| | 424.0 | (2923 MPa) |
|---|---|---|
| | 386.1 | (2662 MPa) |

Deformation (Creep) ASTM D621 (% at 1000 psi (6.9 MPa) load)

In Bar Direction                           0.4

Perpendicular to Bar Direction             0.6

Density g/ml

Gradient column                            0.9595

Infra Red                                  0.957, 0.958

Tensile Properties:

| | In Bar Direction (6" Test Bars) (15mm) (Type I) | Perpendicular to Bar Direction (2-1/2" Test Bars) (64mm) (Type V) |
|---|---|---|
| Tensile Strength, PSI (MPa) | | |
| Yield: | 4743 (32.7) | 4516 (31.1) |
| | 4758 (32.8) | 4526 (31.2) |
| Max: | 4743 (32.7) | 5614 (38.7) |
| | 4758 (32.8) | 5005 (34.5) |
| Break: | 4396 (30.3) | 5004 (34.5) |
| | 3695 (25.5) | 5040 (34.8) |
| Tensile Modulus, KPSI: (MPa) | 611.1 (4213) | 520.3 (3587) |
| | 613.0 (4227) | 513.9 (3543) |
| Elongation, %: | | |
| Break | 355 | 433 |
| | 315 | 400 |

IZOD IMPACT, ft.lb./in. of notch    (Nm/m)

| Bar Direction | Perpendicular to Bar Direction |
|---|---|
| 24.8 (1323) | 26.1 (1393) |
| 22.0 (1174) | 25.0 (1334) |

EXAMPLE 10

Effect of Sequence of Heat-treatment, Cooling, Reheating to a Lower Temperature, and Pressure Recrystallization on UHMWPE.

A UHMW PE bar (3" x 15") (76 x 380mm) of the same type as in Example 1 was heated for five hours at 325°C under $N_2$, then slowly cooled to room temperature. It was reheated to 225°C, and pressure recrystallized as described in Example 9 according to the following schedule:

| 241° to 191°C | 3000 ATM | 2 hours, 15 minutes |
|---|---|---|
| 191° to 181°C | 3000 ATM | 2 hours |
| 181° to 171°C | 3000 ATM | 6 hours |
| (3000 ATM = 300 MPa) | | |

The resulting product was machined into test pieces and analyzed with the following results:

**DSC**

|  | Center of Bar | 1 cm in from Bar Edge |
|---|---|---|
| **m.p., °C** | | |
| 1st heat | 149.3 | 149.1 |
| 2nd heat | 134.3 | 135.2 |
| ΔT | 15 | 13.9 |
| **Heat of Fusion** | | |
| 1st heat | 223.6 J/g | 229.6 J/g |
| 2nd heat | 156.1 J/g | 162.3 J/g |

**Infrared Crystallinity Index**

| In Bar Direction | 0.745 |
|---|---|
| Perpendicular to Bar Direction | 0.759 |

**Tensile Properties**

| Tensile Strength, PSI | (MPa) | | |
|---|---|---|---|
| At Yield | 4706 (32.4) | 4463 (30.1) |
| At Break | 5362 (37.0) | 5326 (36.7) |

13

```
Tensile Modulus,
    KPSI:  (MPa)    649.7 (4480)        404.2 (2787)
Elongation, %
    At Yield         4.7                   4.5
    At Break        330                .  335
Deformation (Creep) ASTM D621 Test
    (% at 1000 psi (6.9 MPa) load)
                                 0.4
                                 0.3
```

Effect of Preheating by Reflux

Alternatively, the preliminary heating of Example 10 may be achieved by refluxing in vapors as described below.

A 3" x 18" (76 x 457mm) rod of UHMWLPE (American Hoechst, Hostalen GUR 415) was heated in refluxing vapors of Krytox®-143AZ (E. I. du Pont de Nemours and Company, Wilmington, Delaware) (at 333-335°C) for 2 hours, 40 minutes. Krytox®-143AZ is a perfluoroalkylpolyether that is a nonflammable, chemically inert liquid having unusually high thermal and oxidative stability. Other materials demonstrating these characteristics may also be suitable. The system was protected by a nitrogen atmosphere and was wrapped with glass insulation to facilitate slow cooling. As compared to the starting material, the resulting product has improved crystallinity (IRCI from 0.27 to 0.47), a tensile modulus (from 210 KPSI to 300 KPSI), and tensile strength at yield (from 3400 to 3850 psi (- 23.4 - 26.5 MPa). Most significantly, the product displays a large increase in elongation at break (from 315% to 893%).

When the above described material was recrystallized from 220°C under 3000 ATM, a new polyethylene resulted possessing extremely high elongation at break (667%) along with the high tensile strength at yield (4900 psi - 33.8 MPa) and the tensile modulus (574 KPSI (3958 MPa)) expected of the superior, enhanced UHMWLPE materials.

| Flex Modulus, KPSI | 436.4<br>431.2<br>433.80 (av) |
|---|---|
| Density | .9684 |
| IZOD IMPACT, (ft.lb./in. of notch) (Nm/m) | 17.1 (913)<br>15.9 (849)<br>16.5 (av) (881) |

EXAMPLE 11

Further evidence shows that UHMWPE polymer pretreated to at least 325°C gives a superior enhanced material (SEUHMW PE).

The purpose of this Example is to show the importance of the preheat temperature.

| Preheat Temperature(C°C) | 225 | 290 | 310 | 325 |
|---|---|---|---|---|
| Crystallinity Index | .553 | .550 | .605 | 0.76 |
| Heat of Fusion, J/g | 186 | 200 | 190 | 219 |
| Tensile, Yield PSI (MPa) | 4268 (29.4) | 4277 (29.5) | 4212 (29.0) | 4819 (33.2) |
| Deformation (Creep) ASTM D621 Test (% at 1000 psi (6.9 MPa) load) | | 0.6 | | 0.4 |
| IZOD ft.lb./in. of Notch (Nm/m) | 20.6 (1100) | 20.7 (1105) | 20.6 (1100) | 24 (1281) |

Effect of Heating Temperature on UHMW PE

The samples were heated by placing 3/4" (19mm) cubes of UHMW polyethylene (Hoechst Hostalen GUR 415, m.w. 4-6 million, fabricated by Westlake) wrapped in Teflon® film in a large test tube protected from air with $N_2$. In the first experiment, a small thermocouple was inserted into the center of the cube, the purpose being to determine the time necessary for the sample to reach test temperature. A plug of glass wool was placed above the sample to control convection currents. The tube was heated with a Wood's metal bath. After the heat treatment was complete, the sample was wrapped in insulation to ensure slow cooling. At a bath temperature of 250°C, the sample required 45 minutes to reach test temperature.

| Time Sample at Temperature hrs:min | Test Temperature °C | Crystallinity Index (by IR) |
|---|---|---|
| 4:00 | 250 | 0.232 |
| 20:00 | 250 | 0.244 |
| 4:00 | 293 | 0.264 |
| :01 | 293 | 0.230 |
| 4:00* | 320-325 | 0.374 |
| 1:00* | 334-335 | 0.378 |
| 1:00* | 340-342 | 0.391 |

*Heated by submerging sample wrapped in Teflon® film under Woods metal as described in the following paragraph.

Effect of Time on Heating UHMW PE

Small cubes (3/4" - 19mm) of UHMW PE cut from the rod form of Hoechst Hostalen GUR 415 were wrapped in Teflon® film, tied to a glass rod, and pushed under the surface of a Wood's metal bath. In the first experiment, a small thermocouple was inserted in the cube. When plunged into a 322-329°C bath, twelve minutes were required for the sample center to reach 321°C. The time at temperature (not the time in the bath) is recorded below. The samples were removed from the bath and wrapped in glass fiber insulation to permit slow cooling which required 1.5 hours to reach 80°C. The extent of change was determined by measuring crystallinity indices.

| Time Sample (at 320-325°C (hrs:min) | Crystallinity Index by (IR) |
|---|---|
| no heating | .258 |
| 0:10 | .261 |
| 0:20 | .294 |
| 1:00 | .330 |
| 4:00 | .374 |

Heat Treatment of UHMW PE (Large Scale)

A 3" diameter by 18" (76 x 457mm) bar of UHMW polyethylene (Hoechst Hostalen GUR 415, m.w. 4-6 million, fabrication by Westlake) was heated under nitrogen at 325°C for 4 hours (65B). The bar was cut into test pieces as was a bar of the same starting polymer that had not been treated. Tests were run sequentially.

|  | Untreated Polymer | Thermally Treated | % Difference |
|---|---|---|---|
| DSC |  | sharper (narrow curve) |  |
| m.p. °C | 139.7 | 137.5 |  |
| Heat of Fusion J/g | 154.6 | 197.5 | +28 |
| Crystallinity Index (IR) | 0.258 | 0.386 | +50 |

Tensile Properties

Tensile Strength, psi (MPa)

| | | | |
|---|---|---|---|
| Yield | 3380 (23.3) | 3694 (25.5) | |
| | 3456 (23.8) | 3642 (25.1) | |
| | 3418 (23.6) (av) | 3668 (25.3) (av) | +7.3 |
| Max. | 5361 (37.0) | 4706 (32.4) | |
| | 4864 (33.5) | 4673 (32.2) | |
| | 5113 (35.3) (av) | 4690 (32.3) (av) | |
| Break | 5361 (37.0) | 4705 (32.4) | |
| | 4864 (33.5) | 4673 (32.2) | |
| | 5113 (35.3) (av) | 4689 (av) | |

Elongation, % Break

| | | |
|---|---|---|
| 330 | 490 | |
| 300 | 500 | |
| 315 (av) | 495 (av) | +57 |

Modulus, KPsi (MPa)

| | | |
|---|---|---|
| 208.4 (1437) | 244.6 (1687) | |
| 210.5 (1451) | 253.7 (1749) | |
| 209.5 (1445) (av) | 249.1 (1718) (av) | +19 |

Flex Modulus, KPsi (MPa)

| | | |
|---|---|---|
| 124.4 (858) | 151.5 (1045) | |
| 137.1 (945) | 146.8 (1012) | |
| 130.7 (901) (av) | 149.1 (1028) (av) | +14 |

IZOD IMPACT, (ft.lb./in. of notch) (Nm/m)

| | | |
|---|---|---|
| 15.93 (850) | 19.97 (1066) | |
| 20.81 (1111) | 22.68 (1211) | |
| 18.37 (981) (av) | 21.32 (1138) (av) | +16 |

Deformation (Creep) ASTM D621 Test
(% at 1000 psi/load) *(6.9MPa)*

|  |  |  |
|---|---|---|
| 1.8 | 1.6 | -17 |
| 1.7 | 1.3 |  |

Similarly, a 3" (76mm) diameter bar of different UHMW polyethylene (Himont 1900, m.w. 1,000,000) was heat pretreated in an inert atmosphere, for example, of $N_2$. The physical properties of the product had greatly inproved elongation and impact resistance.

|  | Untreated Polymer | Thermally Treated | % Difference |
|---|---|---|---|
| **DSC** |  |  |  |
| Heat of Fusion J/g | 166.3 | 190.7 | +15 |
| Crystallinity Index (IR) | .284 | .379 | +33 |

**Tensile Properties**

Tensile Strength, psi (MPa)

| | Untreated | Thermally Treated | % Difference |
|---|---|---|---|
| Yield | 3544 (24.4) | 3721 (25.7) | |
| | 3703 (25.5) | 3589 (24.7) | |
| | 3622 (25.0) (av) | 3655 (25.2) (av) | – 0 |
| **Max.** | 7387 (50.9) | 6545 (45.1) | |
| | 7190 (49.6) | 5999 (41.4) | |
| | 7289 (50.3) (av) | 6272 (43.2) (av) | –14 |

Elongation, % Break

| | Untreated | Thermally Treated | % Difference |
|---|---|---|---|
| | 200 | 343 | |
| | 216 | 293 | |
| | 208 (av) | 318 (av) | +53 |
| **% Yield** | 16.6 | 20 | |
| | 20 | 16.6 | |
| **Modulus, KPsi (MPa)** | 128.4 (885) | 212.7 (1467) | |
| | 216.2 (1491) | 192.7 (1329) | |
| | 202.7 (1398) (av) | 202.7 (1398) (av) | 0 |

## IZOD IMPACT, (ft.lb./in. of notch) (Nm/m)

```
13.05 (697)        24.26 (1295)
11.94 (637)        17.12 (914)
12.49 (656) (av)   21.09 (1126) (av)   +65
```

EXAMPLE 12

A 3" (76mm) diameter bar (rod), 18" (457mm) in length, of American Hoechst Hostalen GUR 415 ultrahigh molecular weight polyethylene, was heated in an oven and then encapsulated with low molecular weight polyethylene by rolling the hot rod onto a 1/16" (1.6mm) sheet of low molecular weight polyethylene heated to 180°C on a large hot plate. An intervening sheet of "Teflon" Polytetrofluoroethylene film was kept on the encapsulated rod to prevent sticking to the hot plate. The rod ends were similarly sealed. The "Teflon" film was kept on the encapsulated rod to prevent sticking in the reactor.

The bar was heated to 225°C under a nitrogen atmosphere and transfered to the reactor at 225°C. After sealing, the reactor pressure was taken to 3000 atmospheres which caused the temperature to reach 237°C. The reactor was permitted to cool to 180°C in 6.5 h, then maintained at this temperature for 1h. The temperature was dropped to 170°C, held at this temperature for 3h, then cooled slowly to 150°C from where it was cooled rapidly.

The rod, which remained coated, was cut and machined into two test pieces (A and B) which gave the following results:

| DSC | SAMPLE | |
|---|---|---|
| | A | B |
| 1st Heat: | | |
| Melt point, °C | 149.1 | 153.7 |
| Heat of Fusion, J/g | 219.8 | 209.5 |
| 2nd heat: | | |
| Melt point, °C | 135.5 | 136.6 |
| Heat of fusion, J/g | 141.2 | 144.9 |
| Crystallinity Index (IR) | 0.566 | 0.567 |
| Tensile Properties: | | |
| Tensile Strength, psi (MPa) | | |
| At Yield | 4149 (28.6) | 4076 (28.1) |
| At Max. | 7448 (51.3) | 8138 (56.1) |
| At Break | 7448 (51.3) | 8138 (56.1) |
| Elongation, % | 323 | 346 |
| Modulus, Kpsi (MPa) | 363.6 (2507) | 358.2 (2470) |
| Creep Deformation, % (D621) | 0.6 | 0.6 |
| IZOD Impact, (ftlb/in. of notch) (Nm/m) | 15.9 (849) | 15.8 (843) |

EXAMPLE 13

A 5.75" (146mm) segment of enhanced ultrahigh molecular weight polyethylene prepared as in Example 5, was skived to two films (A and B), of 11 mil and 5 mil thickness, respectively. The following properties were obtained (averaged from five tests per film sample):

| | SAMPLE | |
|---|---|---|
| | A | B |
| Tensile Properties: | | |
| Tensile Strength, psi (MPa) | | |
| At Yield | 3035 (20.9) | 3108 (21.4) |
| At Max. | 6554 (45.2) | 4083 (28.2) |
| At Break | 6481 (44.7) | 4083 (28.2) |
| At 5% Elongation | 2667 (18.4) | 2705 (18.7) |
| Tensile Modulus, Kpsi (MPa) | 129.7 (894) | 165.6 (1142) |
| Elongation at Break, % | 470 | 237.6 |

The skived films were hot drawn in a tenter frame at 140°C. One piece of the 5 mil film was drawn 6 fold in one direction (C). A second piece of the 5 mil film was drawn 3 fold in both directions (D):

| | SAMPLE | |
|---|---|---|
| | C | D |
| Tensile Strength, psi: (MPa) | | |
| At Yield | 37,819 (261) | 13,720 (95) |
| At Max. | 42,058 (290) | 19,358 (133) |
| At Break | 46,426 (320) | 18,995 (131) |
| Tensile Modulus, Kpsi (MPa) | 93.3 (643) | 94.9 (654) |
| Elongation at Break, % | 56 | 132.4 |
| Thickness, mils ($\mu$m) | 2.6 (66) | 1.6 (41) |

CONTROL B

In this experiment, a product was prepared in the manner of Example 1 of U.S. Patent No. 3,944,536.

A sample of the ultrahigh molecular weight polyethylene used in Example 1 previously described herein, measuring 1/4" x 1/4" x 1/8" (6 x 6 x 3mm) thick was heated between a film of polytetrafluoroethylene on a hot plate at atmospheric pressure and at a temperature of 160°C on the upper side and 270°C on the lower side.

The molten sample was transferred quickly to a hydraulic press at room temperature where the sample was subjected to a pressure of 8 tons (about 250,000 psi or about 16,000 ATM 1600 MPa). It required 1.6 seconds to attain full pressure. The resulting polymer was tested. It displayed an infrared crystallinity index of 0.199. Its melting point was 134.5°C and, when reheated, 134.4°C.

**Claims**

1. An ultrahigh molecular weight linear polyethylene exhibiting a flexural modulus of 250,000-500,000 psi (1724 - 3448 MPa) a tensile stress at yield of 3500-4500 psi (24.1 - 31.0 MPa), a tensile stress at break of 4000-9000 psi (27.6 - 62.1 MPa), a tensile modulus of 300,000-700,00 psi (2069 - 4827 MPa), a notched Izod impact resistance of 12-25 ft. lb. per inch of notch (641 - 1335 Nm/m), a creep at a compression of 1000 psi (6.9 MPa) of less than 1% after 24 hours at a temperature of 23°C and a relative humidity of 50%, the polyethylene having a molecular weight of 400,000-10,000,000, a single crystalline melting point of greater than 144°C, the reduction in said melting point upon reheating being greater than 11°C and an infrared crystallinity index of at least about 0.45.

2. The composition of Claim 1 wherein the tensile modulus is 300,000-600,000 psi (2069 - 4137 MPa), the notched Izod impact resistance is 12-20 ft. lb. per inch of notch (641 - 1068 Nm/m) and the infrared crystallinity index is at least 0.5.

3. The composition of Claim 2 shaped into an article wherein its dimensions are at least one inch by at least one inch (25 x 25mm).

4. The composition of Claim 2 shaped into an article wherein its smallest dimension is at least 0.2 inch (5mm).

5. The composition of Claim 2 wherein its infrared crystallinity index is at least about 0.5.

6. An article consisting essentially of the ultrahigh molecular weight linear polyethylene of Claim 1 exhibiting a flexural modulus of 250,000-500,000 psi (1724 - 3448 MPa), a tensile stress at yield of 3500-4500 psi (24.1 - 31.0 MPa), a tensile stress at break of 4000-9000 psi (27.6 - 62.1 MPa), a tensile modulus of 300,000-700,000 psi (2069 - 4827 MPa), a notched Izod impact resistance of 12-25 ft. lb. per inch of notch (641 - 1335 Nm/m), a creep at a compression of 1000 psi (6.9 MPa) of less than 1% after 24 hours at a temperature of 23°C and a relative humidity of 50%, the polyethylene having a molecular weight of 400,000-10,000,000, a single crystalline melting point of greater than 144°C, the reduction in said melting point upon reheating being greater than 11°C and an infrared crystallinity index of at least about 0.45.

7. The shaped article of Claim 6 wherein the tensile modulus is 300,000-600,000 psi, the notched Izod impact resistance is 12-20 ft. lb. per inch of notch (641 - 1068 Nm/m) and the infrared crystallinity index is at least 0.5.

8. The shaped article of Claim 6 wherein its dimensions are at least one inch by at least one inch (25 x 25mm).

9. The shaped article of Claim 6 wherein its smallest dimension is at least 0.2 inch (5mm).

10. The shaped article of Claim 6 wherein its infrared crystallinity index is at least about 0.5.

11. A process for obtaining the ultrahigh molecular weight linear polyethylene of Claim 1 consisting essentially of the following steps:
   (a) forming said article of an ultrahigh molecular weight linear polyethylene having a molecular weight of 400,000-10,000,000;
   (b) subjecting said article to a fluid under pressure of at least 2800 ATM (280 MPa) and a temperature of 190°C-300°C;
   (c) maintaining the temperature from 190°C-300°C and the pressure of at least 2800 ATM (280 MPa) for at least 0.5 hour;
   (d) reducing the temperature to at least below 160°C-170°C while maintaining the pressure at at least 2800 ATM, the rate of reduction in temperature being such that temperature gradients in the shaped article are substantially avoided; and
   (e) cooling to a temperature of about 130°C or below and releasing the pressure to approximately 1 ATM (0.1 MPa) in a manner such that remelting of said article is prevented.

12. The process of Claim 11 wherein step (a) is performed after step (e) is performed.

13. The process of Claim 11 wherein said fluid is water.

14. The process of Claim 13 wherein said pressure in step (b) is at least 3000 ATM (300 MPa).

15. The process of Claim 13 wherein said temperature in step (b) is 200°C-230°C.

16. The process of Claim 13 wherein the temperature and pressure in step (e) is maintained for at least one hour.

17. The process of Claim 13 wherein the surface of the article is shaved after step (e).

18. The process of Claim 13 wherein the cooling rate in step (d) is no greater than 35°C per hour.

**19.** The process as in Claim 13 wherein the cooling rate in step (d) is no greater than 10 ° C per hour.

**20.** An ultrahigh molecular weight linear polyethylene exhibiting a flexural modulus of 250,000-650,000 psi (1724 - 4481 MPa), a tensile stress at yield of 3500-5400 psi (24.1 - 37.2 MPa), a tensile stress at break of 4000-6000 psi (27.6 - 41.4 MPa), a tensile modulus of 300,000-700,000 psi (2069 - 4827 MPa), a notched Izod impact resistance of 12-25 ft. lb. per inch of notch (641 - 1335 Nm/m), a creep at a compression of 1000 psi (6.9 MPa) of less than 1% after 24 hours at a temperature of 23 ° C and a relative humidity of 50%, the polyethylene having a molecular weight of 400,000-10,000,000, a single crystalline melting point of greater than 144 ° C, the reduction in said melting point upon reheating being greater than 11 ° C and an infrared crystallinity index of at least about 0.45.

**21.** The composition of Claim 20 wherein the tensile modulus is 300,000-650,000 psi (2069 - 4481 MPa), the notched Izod impact resistance is 12-25 ft. lb. per inch of notch (641 - 1335 Nm/m) and the infrared crystallinity index is at least 0.5.

**22.** The composition of Claim 21 shaped into an article wherein its dimensions are at least one inch by at least one inch (25 x 25mm).

**23.** The composition of Claim 21 shaped into an article wherein its smallest dimension is at least 0.2 inch (5mm).

**24.** The composition of Claim 21 wherein its infrared crystallinity index is at least about 0.5.

**25.** An article consisting essentially of the ultrahigh molecular weight linear polyethylene of Claim 20 exhibiting a flexural modulus of 250,000-650,000 psi (1724 - 4481 MPa), a tensile stress at yield of 3500-5400 psi (24.1 - 37.2 MPa), a tensile stress at break 4000-6000 psi (27.6 - 41.4 MPa), a tensile modulus of 300,000-700,000 psi (2069 - 4827 MPa), a notched Izod impact resistance of 12-25 ft. lb. per inch of notch (641 - 1335 Nm/m), a creep at a compression of 1000 psi (6.9 MPa) of less than 1% after 24 hours at a temperature of 23 ° C and a relative humidity of 50%, the polyethylene having a molecular weight of 400,000-10,000,000, a single crystalline melting point of greater than 144 ° C, the reduction in said melting point upon reheating being greater than 11 ° C and an infrared crystallinity index of at least about 0.45.

**26.** The article of Claim 25 wherein the tensile modulus is 300,000-650,000 psi (2069 - 4481 MPa), the notched Izod impact resistance is 12-25 ft. lb. per inch of notch (641 - 1335 Nm/m) and the infrared crystallinity index is at least 0.5.

**27.** The article of Claim 26 wherein its dimensions are at least one inch by at least one inch (25 x 25mm).

**28.** The article of Claim 26 wherein its smallest dimension is at least 0.2 inch (5mm).

**29.** The article of Claim 25 wherein its infrared crystallinity index is at least about 0.5.

**30.** A process for obtaining the article of Claim 25 consisting essentially of the following steps:
(a) forming said article of an ultrahigh molecular weight linear polyethylene having a molecular weight of 400,000-10,000,000;
(b) subjecting said article to a preliminary heat treatment at a temperature of between 320-340 ° C, in an inert atmosphere for at least 0.5 hour;
(c) subjecting said article to a fluid under pressure of at least 2800 ATM (280 MPa) and a temperature of 190 ° C-300 ° C;
(d) maintaining the temperature from 190 ° C-300 ° C and the pressure of at least 2800 ATM (280 MPa) for at least 0.5 hour;
(e) reducing the temperature to at least below 160 ° C-170 ° C while maintaining the pressure at at least 2800 ATM (280 MPa), the rate of reduction in temperature being such that temperature gradients in the shaped article are substantially avoided; and
(f) cooling to a temperature of about 130 ° C or below and releasing the pressure to approximately 1 ATM (0.1 MPa) in a manner such that remelting of said article is prevented.

31. The process of Claim 30 wherein step (a) is performed after step (f) is performed.

32. The process of Claim 30 wherein said fluid is water.

33. The process of Claim 32 wherein said pressure in step (c) is at least 3000 ATM (300 MPa).

34. The process of Claim 32 wherein said temperature in step (c) is 200°C-230°C.

35. The process of Claim 32 wherein the temperature and pressure in step (f) is maintained for at least one hour.

36. The process of Claim 32 wherein the surface of the article is shaved after step (f).

37. The process of Claim 32 wherein the cooling rate in step (e) is no greater than 35°C per hour.

38. The process as in Claim 32 wherein the cooling rate in step (e) is no greater than 10°C per hour.

39. An improved folded chain ultrahigh molecular weight linear polyethylene exhibiting a flexural modulus of 150,000-300,000 psi (1034 - 2069 MPa), a tensile stress at yield of 3500-4000 psi (24.1 - 31.0 MPa), a tensile stress at break of 4000-6000 psi (31.0 - 41.4 MPa), a tensile modulus of 150,000-300,000 psi (1034 - 2069 MPa), a notched Izod impact resistance of 15-25 ft. lb. per inch of notch (801 - 1335 Nm/m), a creep at a compression of 1000 psi (6.9 MPa) of less than 2% after 24 hours at a temperature of 23°C and a relative humidity of 50%, the polyethylene having a molecular weight of 400,000-10,000,000, preferably of at least 1,000,000 and an infrared crystallinity index of at least about 0.35.

40. The composition of Claim 39 wherein the elongation (% at break) is 250-900, the notched Izod impact resistance is 15-20 ft. lb. per inch of notch (801 - 1068 Nm/m).

41. The composition of Claim 39 shaped into an article wherein its dimensions are at least one inch by at least one inch (25 x 25mm).

42. The composition of Claim 39 shaped into an article wherein its smallest dimension is at least 0.2 inch (5mm).

43. An article consisting essentially of the improved folded chain ultrahigh molecular weight linear polyethylene of Claim 39 exhibiting a flexural modulus of 150,000-300,000 psi (1034 - 2069 MPa), a tensile stress at yield of 3500-4000 psi (24.1 - 31.0 MPa), a tensile stress at break of 4000-6000 psi (31.0 - 41.4 MPa), a tensile modulus of 150,000-300,000 psi (1034 - 2069 MPa), a notched Izod impact resistance of 15-25 ft. lb. per inch of notch (641 - 1335 Nm/m), a creep at a compression of 1000 psi of less than 2% after 24 hours at a temperature of 23°C and a relative humidity of 50%, the polyethylene having a molecular weight of 400,000-10,000,000, preferably of at least 1,000,000 and an infrared crystallinity index of at least about 0.35.

44. The article of Claim 43 wherein the elongation (% at break) is 250-900, the notched Izod impact resistance is 15-20 ft. lb. per inch of notch (801 - 1068 Nm/m).

45. The article of Claim 44 wherein its dimensions are at least one inch by at least one inch (25 x 25mm).

46. The article of Claim 44 wherein its smallest dimension is at least 0.2 inch (5mm).

47. A process for obtaining the article of Claim 43 consisting essentially of the following steps:
    (a) forming said article of an ultrahigh molecular weight linear polyethylene having a molecular weight of 400,000-10,000,000;
    (b) subjecting said article to a temperature of 190°C-340°C for at least 0.5 hour in an inert atmosphere; and
    (c) cooling the article non-precipitously to a temperature of about 130°C or below.

**48.** The process of Claim 47 wherein step (a) is performed after step (c) is performed.

**49.** The process of Claim 47 wherein said temperature in step (b) is 320°C-340°C.

**50.** The process of Claim 47 wherein the temperature in step (b) is maintained for at least one hour.

**51.** A process for producing a superior, enhanced ultrahigh molecular weight polyethylene displaying an elongation at break of at least 400% consisting essentially of the following steps:

(a) subjecting an ultrahigh molecular weight linear polyethylene having a molecular weight of 400,000 to 10,000,000 to a preliminary heat treatment of refluxing vapors at a temperature of between 320-340°C, in an inert atmosphere for at least 0.5 hour;

(b) subjecting said article to a fluid under pressure of at least 2800 ATM (280 MPa) and a temperature of 190°C-300°C;

(c) maintaining the temperature from 190°C-300°C and the pressure of at least 2800 ATM (280 MPa) for at least 0.5 hour;

(d) reducing the temperature to at least below 160°C-170°C while maintaining the pressure at at least 2800 ATM (280 MPa), the rate of reduction in temperature being such that temperature gradients in the shaped article are substantially avoided; and

(e) cooling to a temperature of about 130°C or below and releasing the pressure to approximately 1 ATM (0.1 MPa) in a manner such that remelting of said article is prevented.

**52.** The process of Claim 51 wherein said fluid is water.

**53.** The process of Claim 51 wherein said pressure in step (b) is at least 3000 ATM (300 MPa).

**54.** The process of Claim 51 wherein said temperature in step (b) is 200°C-230°C.

**Patentansprüche**

**1.** Lineares Polyethylen mit ultrahohem Molekulargewicht, mit einem Biegemodul von 250 000 bis 500 000 psi (1 724 bis 3 448 MPa), einer Streckspannung von 3 500 bis 4 500 psi (24,1 bis 31,0 MPa), einer Reißfestigkeit von 4 000 bis 9 000 psi (27,6 bis 62,1 MPa), einem Zugmodul von 300 000 bis 700 000 psi (2 069 bis 4 827 MPa), einer Izod-Kerbschlagzähigkeit von 12 bis 25 ft•lb/inch der Kerbe (641 bis 1 335 N•m/m), einem Kriechen bei einer Kompression von 1 000 psi (6,9 MPa) von weniger als 1 % nach 24 h bei einer Temperatur von 23 °C und einer relativen Luftfeuchtigkeit von 50 %, wobei das Polyethylen ein Molekulargewicht von 400 000 bis 10 000 000, einen einzigen Kristallschmelzpunkt von mehr als 144 °C, wobei die Erniedrigung dieses Schmelzpunkts bei erneutem Erhitzen größer als 11 °C ist, und einen Infrarot-Kristallinitäts-Index von wenigstens etwa 0,45 hat.

**2.** Zusammensetzung nach Anspruch 1, worin der Zugmodul 300 000 bis 600 000 psi (2 069 bis 4 137 MPa) beträgt, die Izod-Kerbschlagzähigkeit von 12 bis 20 ft•lb/inch der Kerbe (641 bis 1 068 N•m/m) beträgt und der Infrarot-Kristallinitäts-Index wenigstens 0,5 beträgt.

**3.** Zusammensetzung nach Anspruch 2, zu einem Gegenstand geformt, worin dessen Abmessungen wenigstens 1 inch × 1 inch (25 mm × 25 mm) betragen.

**4.** Zusammensetzung nach Anspruch 2, zu einem Gegenstand geformt, worin dessen kleinste Anmessung wenigstens 0,2 inch (5 mm) beträgt.

**5.** Zusammensetzung nach Anspruch 2, worin deren Infrarot-Kristallinitäts-Index wenigstens etwa 0,5 beträgt.

**6.** Gegenstand, im wesentlichen bestehend aus dem linearen Polyethylen mit ultrahohem Molekulargewicht nach Anspruch 1, mit einem Biegemodul von 250 000 bis 500 000 psi (1 724 bis 3 448 MPa), einer Streckspannung von 3 500 bis 4 500 psi (24,1 bis 31,0 MPa), einer Reißfestigkeit von 4 000 bis 9 000 psi (27,6 bis 62,1 MPa), einem Zugmodul von 300 000 bis 700 000 psi (2 069 bis 4 827 MPa), einer Izod-Kerbschlagzähigkeit von 12 bis 25 ft•lb/inch der Kerbe (641 bis 1 335 N•m/m), einem Kriechen bei einer Kompression von 1 000 psi (6,9 MPa) von weniger als 1 % nach 24 h bei einer Temperatur von 23 °C und einer relativen Luftfeuchtigkeit von 50 %, wobei das Polyethylen ein Molekulargewicht von 400 000 bis 10 000 000, einen einzigen Kristallschmelzpunkt von mehr als 144 °C, wobei die Erniedrigung dieses Schmelzpunkts bei erneutem Erhitzen größer als 11 °C ist, und einen Infrarot-Kristallinitäts-Index von wenigstens etwa 0,45 hat.

7. Geformter Gegenstand nach Anspruch 6, worin der Zugmodul 300 000 bis 600 000 psi (2 069 bis 4 137 MPa) beträgt, die Izod-Kerbschlagzähigkeit von 12 bis 20 ft•lb/inch der Kerbe (641 bis 1 068 N•m/m) beträgt und der Infrarot-Kristallinitäts-Index wenigstens 0,5 beträgt.

8. Geformter Gegenstand nach Anspruch 6, worin dessen Abmessungen wenigstens 1 inch × 1 inch (25 mm × 25 mm) betragen.

9. Geformter Gegenstand nach Anspruch 6, worin dessen kleinste Abmessung wenigstens 0,2 inch (5 mm) beträgt.

10. Geformter Gegenstand nach Anspruch 6, worin dessen Infrarot-Kristallinitäts-Index wenigstens etwa 0,5 beträgt.

11. Verfahren zur Herstellung des linearen Polyethylens mit ultrahohem Molekulargewicht nach Anspruch 1, im wesentlichen bestehend aus den folgenden Schritten:
(a) Bilden des genannten Gegenstandes aus einem linearen Polyethylen mit ultrahohem Molekulargewicht mit einem Molekulargewicht von 400 000 bis 10 000 000;
(b) Einwirkenlassen eines fließfähigen Mediums unter einem Druck von wenigstens 2 800 ATM (280 MPa) bei einer Temperatur von 190 °C bis 300 °C auf den genannten Gegenstand;
(c) Aufrechterhalten der Temperatur von 190 °C bis 300 °C und des Druckes von wenigstens 2 800 ATM (280 MPa) über wenigstens 0,5 h;
(d) Erniedrigen der Temperatur auf wenigstens unterhalb von 160 °C bis 170 °C unter Aufrechterhalten des Druckes von wenigstens 2 800 ATM (280 MPa), wobei die Geschwindigkeit der Temperaturerniedrigung so ist, daß Temperaturgradienten in dem geformten Gegenstand im wesentlichen vermieden werden; und
(e) Abkühlen auf eine Temperatur von etwa 130 °C oder darunter und Druckablassen auf etwa 1 ATM (0,1 MPa) in solcher Weise, daß ein erneutes Schmelzen des Gegenstandes verhindert wird.

12. Verfahren nach Anspruch 11, worin der Schritt (a) durchgeführt wird, nachdem der Schritt (e) durchgeführt worden ist.

13. Verfahren nach Anspruch 11, worin das fließfähige Medium Wasser ist.

14. Verfahren nach Anspruch 13, worin der Druck in Schritt (b) wenigstens 3 000 ATM (300 MPa) beträgt.

15. Verfahren nach Anspruch 13, worin die Temperatur in Schritt (b) 200 °C bis 230 °C beträgt.

16. Verfahren nach Anspruch 13, worin die Temperatur und der Druck in Schritt (e) wenigstens 1 h aufrechterhalten werden.

17. Verfahren nach Anspruch 13, worin die Oberfläche des Gegenstandes nach dem Schritt (e) abgeschabt wird.

18. Verfahren nach Anspruch 13, worin die Geschwindigkeit der Abkühlung in Schritt (d) nicht größer als 35 °C/h ist.

19. Verfahren nach Anspruch 13, worin die Geschwindigkeit der Abkühlung in Schritt (d) nicht größer als 10 °C/h ist.

20. Lineares Polyethylen mit ultrahohem Molekulargewicht, mit einem Biegemodul von 250 000 bis 650 000 psi (1 724 bis 4 481 MPa), einer Streckspannung von 3 500 bis 5 400 psi (24,1 bis 37,2 MPa), einer Reißfestigkeit von 4 000 bis 6 000 psi (27,6 bis 41,4 MPa), einem Zugmodul von 300 000 bis 700 000 psi (2 069 bis 4 827 MPa), einer Izod-Kerbschlagzähigkeit von 12 bis 25 ft•lb/inch der Kerbe (641 bis 1

EP 0 373 800 B1

335 N•m/m), einem Kriechen bei einer Kompression von 1 000 psi (6,9 MPa) von weniger als 1 % nach 24 h bei einer Temperatur von 23 °C und einer relativen Luftfeuchtigkeit von 50 %, wobei das Polyethylen ein Molekulargewicht von 400 000 bis 10 000 000, einen einzigen Kristallschmelzpunkt von mehr als 144 °C, wobei die Erniedrigung dieses Schmelzpunkts bei erneutem Erhitzen größer als 11 °C ist, und einen Infrarot-Kristallinitäts-Index von wenigstens etwa 0,45 hat.

21. Zusammensetzung nach Anspruch 20, worin der Zugmodul 300 000 bis 650 000 psi (2 069 bis 4 481 MPa) beträgt, die Izod-Kerbschlagzähigkeit von 12 bis 25 ft•lb/inch der Kerbe (641 bis 1 335 N•m/m) beträgt und der Infrarot-Kristallinitäts-Index wenigstens 0,5 beträgt.

22. Zusammensetzung nach Anspruch 21, zu einem Gegenstand geformt, worin dessen Abmessungen wenigstens 1 inch × 1 inch (25 mm × 25 mm) betragen.

23. Zusammensetzung nach Anspruch 21, zu einem Gegenstand geformt, worin dessen kleinste Abmessung wenigstens 0,2 inch (5 mm) beträgt.

24. Zusammensetzung nach Anspruch 21, worin deren Infrarot-Kristallinitäts-Index wenigstens etwa 0,5 beträgt.

25. Gegenstand, im wesentlichen bestehend aus dem linearen Polyethylen mit ultrahohem Molekulargewicht nach Anspruch 20, mit einem Biegemodul von 250 000 bis 650 000 psi (1 724 bis 4 481 MPa), einer Streckspannung von 3 500 bis 5 400 psi (24,1 bis 41,4 MPa), einer Reißfestigkeit von 4 000 bis 6 000 psi (27,6 bis 41,4 MPa), einem Zugmodul von 300 000 bis 700 000 psi (2 069 bis 4 827 MPa), einer Izod-Kerbschlagzähigkeit von 12 bis 25 ft•lb/inch der Kerbe (641 bis 1 335 N•m/m), einem Kriechen bei einer Kompression von 1 000 psi (6,9 MPa) von weniger als 1 % nach 24 h bei einer Temperatur von 23 °C und einer relativen Luftfeuchtigkeit von 50 %, wobei das Polyethylen ein Molekulargewicht von 400 000 bis 10 000 000, einen einzigen Kristallschmelzpunkt von mehr als 144 °C, wobei die Erniedrigung dieses Schmelzpunkts bei erneutem Erhitzen größer als 11 °C ist, und einen Infrarot-Kristallinitäts-Index von wenigstens etwa 0,45 hat.

26. Geformter Gegenstand nach Anspruch 25, worin der Zugmodul 300 000 bis 650 000 psi (2 069 bis 4 481 MPa) beträgt, die Izod-Kerbschlagzähigkeit von 12 bis 25 ft•lb/inch der Kerbe (641 bis 1 335 N•m/m) beträgt und der Infrarot-Kristallinitäts-Index wenigstens 0,5 beträgt.

27. Geformter Gegenstand nach Anspruch 26, worin dessen Abmessungen wenigstens 1 inch × 1 inch (25 mm × 25 mm) betragen.

28. Geformter Gegenstand nach Anspruch 26, worin dessen kleinste Abmessung wenigstens 0,2 inch (5 mm) beträgt.

29. Geformter Gegenstand nach Anspruch 25, worin dessen Infrarot-Kristallinitäts-Index wenigstens etwa 0,5 beträgt.

30. Verfahren zur Herstellung des linearen Polyethylens mit ultrahohem Molekulargewicht nach Anspruch 25, im wesentlichen bestehend aus den folgenden Schritten:
    (a) Bilden des genannten Gegenstandes aus einem linearen Polyethylen mit ultrahohem Molekulargewicht mit einem Molekulargewicht von 400 000 bis 10 000 000;
    (b) Einwirkenlassen einer vorherigen Wärmebehandlung von wenigstens etwa 0,5 h bei einer Temperatur zwischen 320 °C und 340 °C in einer inerten Atmosphäre auf den genannten Gegenstand;
    (c) Einwirkenlassen eines fließfähigen Mediums unter einem Druck von wenigstens 2 800 ATM (280 MPa) bei einer Temperatur von 190 °C bis 300 °C auf den genannten Gegenstand;
    (d) Aufrechterhalten der Temperatur von 190 °C bis 300 °C und des Druckes von wenigstens 2 800 ATM (280 MPa) über wenigstens 0,5 h;
    (e) Erniedrigen der Temperatur auf wenigstens unterhalb von 160 °C bis 170 °C unter Aufrechterhalten des Druckes von wenigstens 2 800 ATM (280 MPa), wobei die Geschwindigkeit der Temperaturerniedrigung so ist, daß Temperaturgradienten in dem geformten Gegenstand im wesentlichen vermieden werden; und

26

(f) Abkühlen auf eine Temperatur von etwa 130 °C oder darunter und Druckablassen auf etwa 1 ATM (0,1 MPa) in solcher Weise, daß ein erneutes Schmelzen des Gegenstandes verhindert wird.

31. Verfahren nach Anspruch 30, worin der Schritt (a) durchgeführt wird, nachdem der Schritt (f) durchgeführt worden ist.

32. Verfahren nach Anspruch 30, worin das fließfähige Medium Wasser ist.

33. Verfahren nach Anspruch 32, worin der Druck in Schritt (c) wenigstens 3 000 ATM (300 MPa) beträgt.

34. Verfahren nach Anspruch 32, worin die Temperatur in Schritt (c) 200 °C bis 230 °C beträgt.

35. Verfahren nach Anspruch 32, worin die Temperatur und der Druck in Schritt (f) wenigstens 1 h aufrechterhalten werden.

36. Verfahren nach Anspruch 32, worin die Oberfläche des Gegenstandes nach dem Schritt (f) abgeschabt wird.

37. Verfahren nach Anspruch 32, worin die Geschwindigkeit der Abkühlung in Schritt (e) nicht größer als 35 °C/h ist.

38. Verfahren nach Anspruch 32, worin die Geschwindigkeit der Abkühlung in Schritt (e) nicht größer als 10 °C/h ist.

39. Verbessertes lineares Polyethylen mit gefalteter Kette und mit ultrahohem Molekulargewicht, mit einem Biegemodul von 150 000 bis 300 000 psi (1 034 bis 2 069 MPa) , einer Streckspannung von 3 500 bis 4 000 psi (24,1 bis 27,6 MPa), einer Reißfestigkeit von 4 000 bis 6 000 psi (27,6 bis 41,4 MPa), einem Zugmodul von 150 000 bis 300 000 psi (1 034 bis 2 069 MPa), einer Izod-Kerbschlagzähigkeit von 15 bis 25 ft•lb/inch der Kerbe (801 bis 1 335 N•m/m), einem Kriechen bei einer Kompression von 1 000 psi (6,9 MPa) von weniger als 2 % nach 24 h bei einer Temperatur von 23 °C und einer relativen Luftfeuchtigkeit von 50 %, wobei das Polyethylen ein Molekulargewicht von 400 000 bis 10 000 000, vorzugsweise von wenigstens 1 000 000, und einen Infrarot-Kristallinitäts-Index von wenigstens etwa 0,35 hat.

40. Zusammensetzung nach Anspruch 39, worin die prozentuale Reißdehnung 250 bis 900 beträgt, die Izod-Kerbschlagzähigkeit von 15 bis 20 ft•lb/inch der Kerbe (801 bis 1 068 N•m/m) beträgt.

41. Zusammensetzung nach Anspruch 39, zu einem Gegenstand geformt, worin dessen Abmessungen wenigstens 1 inch × 1 inch (25 mm × 25 mm) betragen.

42. Zusammensetzung nach Anspruch 39, zu einem Gegenstand geformt, worin dessen kleinste Abmessung wenigstens 0,2 inch (5 mm) beträgt.

43. Gegenstand, im wesentlichen bestehend aus dem verbesserten linearen Polyethylen mit gefalteter Kette und mit ultrahohem Molekulargewicht nach Anspruch 39, mit einem Biegemodul von 150 000 bis 300 000 psi (1 034 bis 2 069 MPa), einer Streckspannung von 3 500 bis 4 000 psi (24,1 bis 27,6 MPa), einer Reißfestigkeit von 4 000 bis 6 000 psi (27,6 bis 41,4 MPa), einem Zugmodul von 150 000 bis 300 000 psi (1 034 bis 2 069 MPa), einer Izod-Kerbschlagzähigkeit von 15 bis 25 ft•lb/inch der Kerbe (801 bis 1 335 N•m/m), einem Kriechen bei einer Kompression von 1 000 psi (6,9 MPa) von weniger als 1 % nach 24 h bei einer Temperatur von 23 °C und einer relativen Luftfeuchtigkeit von 50 %, wobei das Polyethylen ein Molekulargewicht von 400 000 bis 10 000 000, vorzugsweise von wenigstens 1 000 000, und einen Infrarot-Kristallinitäts-Index von wenigstens etwa 0,35 hat.

44. Geformter Gegenstand nach Anspruch 43, worin die prozentuale Reißdehnung 250 bis 900 beträgt, die Izod-Kerbschlagzähigkeit von 15 bis 20 ft•lb/inch der Kerbe (801 bis 1 068 N•m/m) beträgt.

45. Geformter Gegenstand nach Anspruch 44, worin dessen Abmessungen wenigstens 1 inch × 1 inch (25 mm × 25 mm) betragen.

**46.** Geformter Gegenstand nach Anspruch 44, worin dessen kleinste Abmessung wenigstens 0,2 inch (5 mm) beträgt.

**47.** Verfahren zur Herstellung des Gegenstandes nach Anspruch 43, im wesentlichen bestehend aus den folgenden Schritten:

(a) Bilden des genannten Gegenstandes aus einem linearen Polyethylen mit ultrahohem Molekulargewicht mit einem Molekulargewicht von 400 000 bis 10 000 000;

(b) Einwirkenlassen einer Temperatur von 190 °C bis 340 °C in einer inerten Atmosphäre auf den Gegenstand über wenigstens 0,5 h;

(c) nicht-überstürztes Abkühlenlassen des Gegenstandes auf eine Temperatur von etwa 130 °C oder darunter.

**48.** Verfahren nach Anspruch 47, worin der Schritt (a) durchgeführt wird, nachdem der Schritt (c) durchgeführt worden ist.

**49.** Verfahren nach Anspruch 47, worin die Temperatur in Schritt (b) 320 °C bis 340 °C beträgt.

**50.** Verfahren nach Anspruch 47, worin die Temperatur in Schritt (b) wenigstens 1 h aufrechterhalten wird.

**51.** Verfahren zur Herstellung eines überlegenen, verstärkten Polyethylens mit ultrahohem Molekulargewicht und mit einer Reißdehnung von wenigstens 400 %, im wesentlichen bestehend aus den folgenden Schritten:

(a) Einwirkenlassen einer vorherigen Wärmebehandlung von wenigstens etwa 0,5 h durch zurückfließende Dämpfe bei einer Temperatur zwischen 320 °C und 340 °C in einer inerten Atmosphäre auf das lineare Polyethylen mit ultrahohem Molekulargewicht mit einem Molekulargewicht von 400 000 bis 10 000 000;

(b) Einwirkenlassen eines fließfähigen Mediums unter einem Druck von wenigstens 2 800 ATM (280 MPa) bei einer Temperatur von 190 °C bis 300 °C auf den genannten Gegenstand;

(c) Aufrechterhalten der Temperatur von 190 °C bis 300 °C und des Druckes von wenigstens 2 800 ATM (280 MPa) über wenigstens 0,5 h;

(d) Erniedrigen der Temperatur auf wenigstens unterhalb von 160 °C bis 170 °C unter Aufrechterhalten des Druckes von wenigstens 2 800 ATM (280 MPa), wobei die Geschwindigkeit der Temperaturerniedrigung so ist, daß Temperaturgradienten in dem geformten Gegenstand im wesentlichen vermieden werden; und

(e) Abkühlen auf eine Temperatur von etwa 130 °C oder darunter und Druckablassen auf etwa 1 ATM (0,1 MPa) in solcher Weise, daß ein erneutes Schmelzen des Gegenstandes verhindert wird.

**52.** Verfahren nach Anspruch 51, worin das fließfähige Medium Wasser ist.

**53.** Verfahren nach Anspruch 51, worin der Druck in Schritt (c) wenigstens 3 000 ATM (300 MPa) beträgt.

**54.** Verfahren nach Anspruch 51, worin die Temperatur in Schritt (b) 200 °C bis 230 °C beträgt.

**Revendications**

**1.** Un polyéthylène linéaire à poids moléculaire ultra-élevé présentant un module en flexion de 1724 à 3448 MPa, une limite d'élasticité à la traction de 24,1 à 31,0 MPa, une limite de rupture à la traction de 27,6 à 62,1 MPa, un module en traction de 2069 à 4827 MPa, une résistance au choc Izod avec entaille de 641 à 1335 nm/m d'entaille, un fluage sous une compression de 6,9 MPa inférieur à 1 % après 24 heures à une température de 23°C et une humidité relative de 50 %, le polyéthylène ayant un poids moléculaire de 400 000 à 10 000 000, un seul point de fusion cristalline supérieur à 144°C, l'abaissement dudit point de fusion par réchauffage étant supérieur à 11°C, et un indice de cristallinité par analyse aux infrarouges d'au moins environ 0,45.

**2.** La composition de la revendication 1, dans laquelle le module en traction est de 2069 à 4137 MPa, la résistance au choc Izod avec entaille est de 641 à 1068 nm/m d'entaille et l'indice de cristallinité par analyse aux infrarouges est d'au moins 0,5.

**3.** La composition de la revendication 2, façonnée en un article dont les dimensions sont d'au moins 25 mm sur au moins 25 mm.

**4.** La composition de la revendication 2, façonnée en un article dont la plus petite dimension est d'au moins 5 mm.

**5.** La composition de la revendication 2, dont l'indice de cristallinité par analyse aux infrarouges est d'au moins environ 0,5.

**6.** Un article constitué essentiellement du polyéthylène linéaire à poids moléculaire ultra-élevé de la revendication 1, présentant un module en flexion de 1724 à 3448 MPa, une limite d'élasticité à la traction de 24,1 à 31,0 MPa, une limite de rupture à la traction de 27,6 à 62,1 MPa, un module en traction de 2069 à 4827 MPa, une résistance au choc Izod avec entaille de 641 à 1335 nm/m d'entaille, un fluage sous une compression de 6,9 MPa inférieur à 1 % après 24 heures à une température de 23 °C et une humidité relative de 50 %, le polyéthylène ayant un poids moléculaire de 400 000 à 10 000 000, un seul point de fusion cristalline supérieur à 144 °C, l'abaissement dudit point de fusion par réchauffage étant supérieur à 11 °C, et un indice de cristallinité par analyse aux infrarouges d'au moins environ 0,45.

**7.** L'article façonné de la revendication 6, dans lequel le module en traction est de 2069 à 4137 MPa, la résistance au choc Izod avec entaille est de 641 à 1068 nm/m d'entaille et l'indice de cristallinité par analyse aux infrarouges est d'au moins 0,5.

**8.** L'article façonné de la revendication 6, dont les dimensions sont d'au moins 25 mm sur au moins 25 mm.

**9.** L'article façonné de la revendication 6, dont la plus petite dimension est d'au moins 5 mm.

**10.** L'article façonné de la revendication 6, dont l'indice de cristallinité par analyse aux infrarouges est d'au moins environ 0,5.

**11.** Un procédé pour obtenir le polyéthylène linéaire à poids moléculaire ultra-élevé de la revendication 1, qui se compose essentiellement des étapes suivantes :
(a) former ledit article constitué d'un polyéthylène linéaire à poids moléculaire ultra-élevé ayant un poids moléculaire de 400 000 à 10 000 000 ;
(b) soumettre ledit article à un fluide sous une pression d'au moins 280 MPa et à une température de 190 °C à 300 °C ;
(c) maintenir la température de 190 °C à 300 °C et la pression d'au moins 280 MPa pendant au moins 0,5 heure ;
(d) abaisser la température au moins jusqu'au-dessous de 160 °C à 170 °C tout en maintenant la pression à au moins 280 MPa, la vitesse d'abaissement de la température étant telle que des gradients de température dans l'article façonné soient sensiblement évités ; et
(e) refroidir jusqu'à une température d'environ 130 °C ou moins et relâcher la pression jusqu'à environ 0,1 MPa de telle manière que soit empêchée une refonte dudit article.

**12.** Le procédé de la revendication 11, dans lequel l'étape (a) est exécutée après l'exécution de l'étape (e).

**13.** Le procédé de la revendication 11, dans lequel ledit fluide est l'eau.

**14.** Le procédé de la revendication 13, dans lequel ladite pression dans l'étape (b) est d'au moins 300 MPa.

**15.** Le procédé de la revendication 13, dans lequel ladite température dans l'étape (b) est de 200 °C à 230 °C.

**16.** Le procédé de la revendication 13, dans lequel la température et la pression dans l'étape (e) sont maintenues pendant au moins une heure.

**17.** Le procédé de la revendication 13, dans lequel la surface de l'article est pelée après l'étape (e).

**18.** Le procédé de la revendication 13, dans lequel la vitesse de refroidissement dans l'étape (d) n'est pas supérieure à 35°C par heure.

**19.** Le procédé de la revendication 13, dans lequel la vitesse de refroidissement dans l'étape (d) n'est pas supérieure à 10°C par heure.

**20.** Un polyéthylène linéaire à poids moléculaire ultra-élevé présentant un module en flexion de 1724 à 4481 MPa, une limite d'élasticité à la traction de 24,1 à 37,2 MPa, une limite de rupture à la traction de 27,6 à 41,4 MPa, un module en traction de 2069 à 4827 MPa, une résistance au choc Izod avec entaille de 641 à 1335 nm/m d'entaille, un fluage sous une compression de 6,9 MPa inférieur à 1 % après 24 heures à une température de 23°C et une humidité relative de 50 %, le polyéthylène ayant un poids moléculaire de 400 000 à 10 000 000, un seul point de fusion cristalline supérieur à 144°C, l'abaissement dudit point de fusion par réchauffage étant supérieur à 11°C, et un indice de cristallinité par analyse aux infrarouges d'au moins environ 0,45.

**21.** La composition de la revendication 20, dans laquelle le module en traction est de 2069 à 4481 MPa, la résistance au choc Izod avec entaille est de 641 à 1335 nm/m d'entaille et l'indice de cristallinité par analyse aux infrarouges est d'au moins 0,5.

**22.** La composition de la revendication 21, façonnée en un article dont les dimensions sont d'au moins 25 mm sur au moins 25 mm.

**23.** La composition de la revendication 21, façonnée en un article dont la plus petite dimension est d'au moins 5 mm.

**24.** La composition de la revendication 21, dont l'indice de cristallinité par analyse aux infrarouges est d'au moins environ 0,5.

**25.** Un article constitué essentiellement du polyéthylène linéaire à poids moléculaire ultra-élevé de la revendication 20, présentant un module en flexion de 1724 à 4481 MPa, une limite d'élasticité à la traction de 24,1 à 37,2 MPa, une limite de rupture à la traction de 27,6 à 41,4 MPa, un module en traction de 2069 à 4827 MPa, une résistance au choc Izod avec entaille de 641 à 1335 nm/m d'entaille, un fluage sous une compression de 6,9 MPa inférieur à 1 % après 24 heures à une température de 23°C et une humidité relative de 50 %, le polyéthylène ayant un poids moléculaire de 400 000 à 10 000 000, un seul point de fusion cristalline supérieur à 144°C, l'abaissement dudit point de fusion par réchauffage étant supérieur à 11°C, et un indice de cristallinité par analyse aux infrarouges d'au moins environ 0,45.

**26.** L'article de la revendication 25, dans lequel le module en traction est de 2069 à 4481 MPa, la résistance au choc Izod avec entaille est de 641 à 1335 nm/m d'entaille et l'indice de cristallinité par analyse aux infrarouges est d'au moins 0,5.

**27.** L'article de la revendication 26, dont les dimensions sont d'au moins 25 mm sur au moins 25 mm.

**28.** L'article de la revendication 26, dont la plus petite dimension est d'au moins 5 mm.

**29.** L'article de la revendication 25, dont l'indice de cristallinité par analyse aux infrarouges est d'au moins environ 0,5.

**30.** Un procédé pour obtenir l'article de la revendication 25, qui se compose essentiellement des étapes suivantes :
(a) former ledit article constitué d'un polyéthylène linéaire à poids moléculaire ultra-élevé ayant un poids moléculaire de 400 000 à 10 000 000 ;
(b) soumettre ledit article à un traitement thermique préliminaire à une température comprise entre 320 et 340°C dans une atmosphère inerte pendant au moins 0,5 heure ;

(c) soumettre ledit article à un fluide sous une pression d'au moins 280 MPa et à une température de 190 °C à 300 °C ;

(d) maintenir la température de 190 °C à 300 °C et la pression d'au moins 280 MPa pendant au moins 0,5 heure ;

(e) abaisser la température au moins jusqu'au-dessous de 160 °C à 170 °C tout en maintenant la pression à au moins 280 MPa, la vitesse d'abaissement de la température étant telle que des gradients de température dans l'article façonné soient sensiblement évités ; et

(f) refroidir jusqu'à une température d'environ 130 °C ou moins et relâcher la pression jusqu'à environ 0,1 MPa de telle manière que soit empêchée une refonte dudit article.

31. Le procédé de la revendication 30, dans lequel l'étape (a) est exécutée après l'exécution de l'étape (f).

32. Le procédé de la revendication 30, dans lequel ledit fluide est l'eau.

33. Le procédé de la revendication 32, dans lequel ladite pression dans l'étape (c) est d'au moins 300 MPa.

34. Le procédé de la revendication 32, dans lequel ladite température dans l'étape (c) est de 200 °C à 230 °C.

35. Le procédé de la revendication 32, dans lequel la température et la pression dans l'étape (f) sont maintenues pendant au moins une heure.

36. Le procédé de la revendication 32, dans lequel la surface de l'article est pelée après l'étape (f).

37. Le procédé de la revendication 32, dans lequel la vitesse de refroidissement dans l'étape (e) n'est pas supérieure à 35 °C par heure.

38. Le procédé de la revendication 32, dans lequel la vitesse de refroidissement dans l'étape (e) n'est pas supérieure à 10 °C par heure.

39. Un polyéthylène linéaire à poids moléculaire ultra-élevé à chaîne repliée amélioré présentant un module en flexion de 1034 à 2069 MPa, une limite d'élasticité à la traction de 24,1 à 31,0 MPa, une limite de rupture à la traction de 31,0 à 41,4 MPa, un module en traction de 1034 à 2069 MPa, une résistance au choc Izod avec entaille de 801 à 1335 nm/m d'entaille, un fluage sous une compression de 6,9 MPa inférieur à 2 % après 24 heures à une température de 23 °C et une humidité relative de 50 %, le polyéthylène ayant un poids moléculaire de 400 000 à 10 000 000, de préférence d'au moins 1 000 000, et un indice de cristallinité par analyse aux infrarouges d'au moins environ 0,35.

40. La composition de la revendication 39, dans laquelle l'allongement (% à la rupture) est de 250 à 900, la résistance au choc Izod avec entaille est de 801 à 1068 nm/m d'entaille.

41. La composition de la revendication 39, façonnée en un article dont les dimensions sont d'au moins 25 mm sur au moins 25 mm.

42. La composition de la revendication 39, façonnée en un article dont la plus petite dimension est d'au moins 5 mm.

43. Un article constitué essentiellement du polyéthylène linéaire à poids moléculaire ultra-élevé à chaîne repliée amélioré de la revendication 39, présentant un module en flexion de 1034 à 2069 MPa, une limite d'élasticité à la traction de 24,1 à 31,0 MPa, une limite de rupture à la traction de 31,0 à 41,4 MPa, un module en traction de 1034 à 2069 MPa, une résistance au choc Izod avec entaille de 801 à 1335 nm/m d'entaille, un fluage sous une compression de 6,9 MPa inférieur à 2 % après 24 heures à une température de 23 °C et une humidité relative de 50 %, le polyéthylène ayant un poids moléculaire de 400 000 à 10 000 000, de préférence d'au moins 1 000 000, et un indice de cristallinité par analyse aux infrarouges d'au moins environ 0,35.

**44.** L'article de la revendication 43, dans lequel l'allongement (% à la rupture) est de 250 à 900, la résistance au choc Izod avec entaille est de 801 à 1068 nm/m d'entaille.

**45.** L'article de la revendication 44, dont les dimensions sont d'au moins 25 mm sur au moins 25 mm.

**46.** L'article de la revendication 44, dont la plus petite dimension est d'au moins 5 mm.

**47.** Un procédé pour obtenir l'article de la revendication 43, qui se compose essentiellement des étapes suivantes :
   (a) former ledit article constitué d'un polyéthylène linéaire à poids moléculaire ultra-élevé ayant un poids moléculaire de 400 000-10 000 000 ;
   (b) soumettre ledit article à une température de 190°C à 340°C pendant au moins 0,5 heure dans une atmosphère inerte ; et
   (c) refroidir l'article sans hâte jusqu'à une température d'environ 130°C ou moins.

**48.** Le procédé de la revendication 47, dans lequel l'étape (a) est exécutée après l'exécution de l'étape (c).

**49.** Le procédé de la revendication 47, dans lequel ladite température dans l'étape (b) est de 320°C à 340°C.

**50.** Le procédé de la revendication 47, dans lequel la température dans l'étape (b) est maintenue pendant au moins une heure.

**51.** Un procédé pour produire un polyéthylène à poids moléculaire ultra-élevé amélioré, supérieur, présentant un allongement à la rupture d'au moins 400 %, qui se compose essentiellement des étapes suivantes :
   (a) soumettre un polyéthylène linéaire à poids moléculaire ultra-élevé ayant un poids moléculaire de 400 000 à 10 000 000 à un traitement thermique préliminaire par des vapeurs au reflux à une température comprise entre 320 et 340°C dans une atmosphère inerte pendant au moins 0,5 heure ;
   (b) soumettre ledit article à un fluide sous une pression d'au moins 280 MPa et à une température de 190°C à 300°C ;
   (c) maintenir la température de 190°C à 300°C et la pression d'au moins 280 MPa pendant au moins 0,5 heure ;
   (d) abaisser la température au moins jusqu'au-dessous de 160°C à 170°C tout en maintenant la pression à au moins 280 MPa, la vitesse d'abaissement de la température étant telle que des gradients de température dans l'article façonné soient sensiblement évités ; et
   (e) refroidir jusqu'à une température d'environ 130°C ou moins et relâcher la pression jusqu'à environ 0,1 MPa de telle manière que soit empêchée une refonte dudit article.

**52.** Le procédé de la revendication 51, dans lequel ledit fluide est l'eau.

**53.** Le procédé de la revendication 51, dans lequel ladite pression dans l'étape (b) est d'au moins 300 MPa.

**54.** Le procédé de la revendication 51, dans lequel ladite température dans l'étape (b) est de 200°C à 230°C.

FIGURE 1

EP 0 373 800 B1

FIGURE 2